# EUROPEAN PATENT APPLICATION

(11) **EP 1 205 194 A1**
(43) Date of publication of application: **15.05.2002**
(21) Application number: 00870263.1
(22) Date of filing: 07.11.2000
(51) Int. Cl.: A61L 9/03, A01M 1/20

(54) **Self-heating device for prolonged dispensing of volatilized materials**

(71) Applicant: THE PROCTER & GAMBLE COMPANY, Cincinnati, Ohio 45202 (US)
(72) Inventor: Blenkiron, Peter, Egham, Surrey TW20 8XB (GB); Burrowes, Lee, Woking, Surrey GU21 4PR (GB); Curtis, Terence Graham, High Wycombe, Bucks HP11 1JN (GB); Metzger-Groom, Sabine Ursula, Morpeth, Northumberland NE61 2DJ (GB)
(74) Representative: Canonici, Jean-Jacques

(57) **Abstract**

Disclosed are devices for dispensing volatilized materials such as fragrances into the atmosphere surrounding each such device. Release of volatilized materials from a volatilization zone in such a device is promoted by the generation of heat via the non-combustive oxidation reaction of thermogenic materials held within one or more heating zones in the device. The device is activated by exposing both the volatilization zone and the heating zone(s) to the atmosphere surrounding the device. Thermogenic materials used to generate thermal energy are held in at least two different regions which have relatively faster and relative slower heat production rates, respectively. In this manner, devices are provided which are quick to heat up to an acceptable volatilization temperature but which are also provide acceptably prolonged heat generation to keep the device dispensing volatilized materials.

## Description

### Technical Field/Field of the Invention

The present invention relates to a self-heating device for dispensing volatile materials, such as fragrances, into the atmosphere surrounding the device for a relatively prolonged period of time.

### Background of the Invention

Devices for providing and dispensing volatile materials are well-known in the art. Such devices can include room freshners or fragrancing articles, vaporizers for humidification or dispensing and dispersal of therapeutic vapors, incense sticks, fragrancing or insect-repelling candles, and the like. Such devices generally involve utilization of some source of heat which promotes the volatilization of the materials to be dispensed and, of course, some source of the volatile or volatilizable materials themselves.

Some fragrancing devices, such as candles and incense sticks, involve use of an open flame or an active combustion reaction to provide the source of heat which promotes volatilization. Besides the obvious drawbacks of the hazards of using such types of heat sources, arrangements which involve flames or combustion are not especially portable and cannot be used, for example, in automobiles or around flammable materials.

Other types of dispensing devices for volatiles use electrical energy as a heat source. Plug-in room air-fresheners, for example, are commercially marketed under the tradenames *Ambi-Pur* and *Glade.* The amount of electrical energy required to operate devices of this type renders such devices relatively non-portable.

Some articles and devices for emitting or dispensing volatile materials employ non-combustive thermogenic materials as a heat source useful for promoting volatilization of the ingredients to be emitted or dispensed. Villamarin; PCT Patent Application WO 91/07996; Published June 13, 1991, for example, discloses a self-contained fragrance delivery package, comprising a fluent volatilizable fragrance material and an air-activatable chemical heat source. Disclosed configurations include a layered pad with covered perforations which can be removed to expose the heat source to air and activate the pad. Other dispensing devices using a non-combustive chemical heat source are disclosed in Takei; U.S. Patent 4,330,506; Issued May 18, 1982; Koiso et al; U.S. Patent 4,516,564; Issued May 14, 1985 and Arao; Japanese Patent Application 63175771; Published January 26, 1990.

Such devices in general are not necessarily readily consumer activatable. Furthermore, some devices of this type may have problems with acceptably rapid transfer of heat to the volatilizable materials or problems with maintaining such heat transfer, and hence device activation, over an acceptably long period of time. For example, devices of this general type which do rapidly heat up and begin emitting volatilized materials quickly may not be able to generate heat at a rate to acceptably continue volatilizing materials to be dispensed or emitted for more than 30 to 60 minutes. Devices which are configured to generate volatilizing thermal energy over longer periods of time may not generate enough heat initially upon device activation to begin immediate volatilization.

Given the foregoing situation, there is a continuing need to provide dispensing devices in forms which are safe, portable and readily consumer activatable. There is further a need for such devices which dispense the desired volatile materials very soon after device activation and in suitable amounts over an acceptably prolonged period of time.

### Summary of the Invention

The present invention is directed to a dispensing device for emitting volatile materials into the atmosphere surrounding the device. The invention further relates to the use of such a device for emitting volatiles such as fragrances.

In the broadest sense, the devices herein comprise at least one volatilization zone containing volatilizable materials and at least one heating zone containing oxygen-activatable thermogenic materials. The volatilization zone is openable to the outside atmosphere to permit emission of volatilized materials therefrom and is juxtaposed with and in thermodynamic communication with the heating zone(s).

The thermogenic materials in the heating zone(s) are capable of producing thermal energy via a non-combustive reaction when contacted with oxygen-containing gas. Such materials are also capable of transferring the thermal energy they produce to the volatilization zone to thereby volatilize the volatilizable material in that zone. Within the thermogenic materials in the heating zone(s), there are at least two distinct regions. Upon activation of the device, thermal energy in one such region of the thermogenic materials is produced at a faster rate and for a shorter period of time than thermal energy is produced in at least one other region of the thermogenic materials.

In one preferred configuration of the dispensing devices herein, the thermogenic materials are placed in both a first heating zone and a second heating zone. The device then further comprises means, such as openable apertures, for introducing oxygen-containing gas into both the first and second heating zones at introduction rates such that thermal energy is generated more rapidly and for a shorter period of time in the first heating zone than in the second heating zone. In an especially preferred embodiment of this device configuration, the first and second heating zones of the device both include openable apertures which permit airflow into both heating zones. However, the total cross-sectional area of the apertures in the first heating zone is from 10% to 700% larger than the total cross-sectional area of the apertures in the second heating zone.

In a highly preferred configuration, the dispensing devices herein comprise an insert, which itself comprises three separate zones, and a holder for this insert. One zone of the insert is a substantially rigid volatilization zone which contains volatilizable materials. The other two zones of the insert are first and second heating zones which contain air-activatable thermogenic materials. This volatilization zone and the two heating zones are juxtaposed and in thermodynamic communication with each other within the insert.

Both the volatilization zone and the heating zones of the insert are sealed from the atmosphere surrounding the insert by rupturable sealing means such as, for example, aluminum foil. Such sealing means can be ruptured to expose all three zones to the atmosphere. Such exposure activates the thermogenic materials within the heating zone and releases the volatile materials from the volatilization zone into the atmosphere.

In this highly preferred device configuration, the holder for the insert comprises both a substantially rigid base and a substantially rigid cover for the base. The base and/or the cover of the holder will also comprise means, such as relatively sharp protrusions, which can be used to rupture the seals of the zones of the insert. These rupturing means can be activated by the user of the dispensing device to rupture the insert seals, and this serves to activate the dispensing device. Upon activation, the heat generated in the heating zones enhance volatilization of the materials in the volatilization zone and promotes their emission into the surrounding atmosphere. The sealing means of the first and second heating zones of the insert are configured such that the opening, e.g., rupturing, of these sealing means results in air introduction into the first heating zone at a faster rate than the rate of air introduction into the second heating zone.

In all of these different device configurations and embodiments, the differing rates of thermal energy production within different regions of the thermogenic materials, e.g., in the different heating zones, result in devices with both especially desirable initial heat up and prolonged activity. Such devices heat up and emit volatilized material quickly by virtue of the faster energy production and higher temperature produced in the first region or zone, but they also are active for an extended period of time by virtue of the slower energy production in the second region or zone.

### Brief Description of the Drawings

Figure 1 is a view in perspective of a fragrance dispensing device according to the present invention, prior to the activation of the device.

Figure 2 is a view in perspective of a fragrance dispensing device according to the present invention after the device has been activated.

Figure 3 is an exploded view of a device according to the present invention showing the holder and the insert of the device.

### Detailed Description of the Invention

The dispensing device of the present invention comprises both volatilization and heating zones. These zones are preferably found within an insert which is carried and held within a two-part holder. All of the elements of the various dispensing device configurations herein are described in detail as follows :

### A) Insert

An insert is the component of the preferred dispensing device which holds both the volatilizable materials to be emitted from the device and the thermogenic materials which serve to provide the thermal energy to help volatilize such materials. Both the volatilizable materials which are held within at least one volatilization zone and the thermogenic materials which are held within one or more heating zones are sealed from the atmosphere surrounding the insert until the device is activated by rupturing the seals of these zones and exposing the contents of the insert to the outside air.

### i) Volatilization Zone

One essential component of the devices, and preferably the insert, herein is the volatilization zone. It is the volatilization which holds the volatilizable materials which the dispensing device of this invention is designed to emit. The volatilization zone can be of any shape or configuration but will generally be substantially rigid.

Preferably the volatilization zone will have a volume of from 1 cm³ to 50 cm³, more preferably from 3 cm³ to 15 cm³. It will also preferably be elongated, having a major dimension of from 1 cm to 15 cm, more preferably from 2 cm to 10 cm. Preferably the volatilization zone will be configured within the device, e.g., within the insert, such that the major dimension of the volatilization zone is generally in a vertical position as the insert is held within the dispensing device.

Frequently the volatilization zone, e.g., within the insert, will be generally cylindrical in configuration. Alternatively, the volatilization zone can have a tapered configuration. Preferably the volatilization zone will be openable to the atmosphere at both of its ends. Concurrently filed European Patent Application No. ___________ (P&G Case No. CM-2467F) is directed to devices having a volatilization zone of this configuration

In the cylindrical configuration, the volatilization zone will typically have a circular cross-sectional area which ranges from 1 cm² to 10 cm² more preferably from 2 cm² to 6 cm². In the tapered configuration, the volatilization zone will generally have a smaller circular cross-sectional area at or near the top of the zone than at or near the bottom of the zone. For the tapered configuration, the circular cross-sectional area at or near the top of the volatilization zone will typically range from 0.5 cm² to 5 cm², more preferably from 1 cm² to 3 cm², and the circular cross-sectional area at or near the bottom will typically range from 1 cm² to 10 cm², more preferably from 2 cm² to 6 cm². In the tapered configuration, the volatile or volatilizable materials tend to move through the volatilization zone faster at or near the top by virtue of the effect of the Bernulli principle. This helps to boost the effectiveness of the volatilized material emission into the atmosphere surrounding the device.

The volatilization zone, as with the rest of the elements of the dispensing device herein, can be made of any suitable material which provides the requisite rigidity, strength, heat resistance/heat conductivity and compatibility with the materials to be held therein. Typically thermoplastic or thermosetting materials such as polyethylene, polypropylene, polystyrene, acrylic resins, and the like, may be utilized to form the volatilization zone, e.g., within the insert.

The volatilization zone will contain the volatilizable materials which are to be emitted from the device upon rupturing of the volatilization zone sealing means and exposure of this zone to this surrounding atmosphere. For purposes of this invention, the term "volatilizable materials" will include materials which may already be largely in the vapor phase at ambient temperature (e.g. 20°C). Volatilizable materials also include those which may be completely or partially in a liquid or solid phase at ambient temperature but which can be vaporized by the heat generated by the thermogenic materials in the heating zone(s) of the devices herein.

Suitable volatilizable materials are those which can act as fragrances, insect repellents, insecticides and materials which are or which produce therapeutic vapors. Preferred volatilizable materials are fragrances which can include any odoriferous materials having a vapor pressure of at least 17.5 mmHg @ 20°C. Generally, the devices herein will contain from 0.1 gram to 10 grams, more preferably from 0.5 gram to 3 grams, most preferably from 1 gram to 2 grams of the volatilizable materials sealed until activation within the volatilization zone.

The volatilizable materials may be placed into the sealed volatilization chamber in neat form, or they may be held in the volatilization zone in combination with a liquid or solid carrier substrate. Any material which will absorb, adsorb, or otherwise hold or carry the volatilizable material until the volatilization zone is exposed to the atmosphere can be employed as carrier or carrier substrate. Especially suitable carriers can include solid absorbent materials such as felt or cellulosics including paper, cotton, airfelt, and the like. Other carrier include porous polyolefin materials. An especially preferred carrier for materials to be held in the volatilization zone is the porous polypropylene material marketed under the tradename, Accurel™.

### ii) Heating Zone(s)

The volatilization zone herein is juxtaposed and in thermodynamic communication with at least one heating zone containing oxygen-activatable thermogenic materials. The heating zone(s), like the volatilization zone, can be constructed of any suitable material which will hold thermogenic materials and be compatible with such materials and the heat generated thereby when the devices herein are activated. Preferably the heating zone(s) will have a volume of 10 cm³ to 100 cm³, more preferably from 20 cm³ to 40 cm³.

In preferred embodiments wherein the volatilization zone is generally cylindrical, the heating zone(s) will be of generally annular configuration, surrounding the volatilization zone, with the generally cylindrical volatilization zone running through the core of the heating zone(s).

The heating zone(s) will contain thermogenic materials which produce thermal energy via a non-combustive oxidation reaction upon contact with oxygen-containing gas such as for example upon exposure to and contact with air. Typically such materials are those used to bring about the exothermic oxidation of iron and are used in particulate form. Generally the heating zone(s) will contain from 10 grams to 50 grams, more preferably from 15 grams to 30 grams, of the thermogenic material combination.

A preferred combination of air-activatable thermogenic materials comprises from 30% to 80% by weight of the combination of iron powder, from 3% to 25% by weight of the combination of carbon particles, from 0.5% to 10% by weight of the combination of a metal salt such as alkali metal or alkaline earth metal salts, and from 1% to 40% by weight of the combination of water. Such thermogenic material combinations may also contain an additional water-holding particles (e.g., vermiculite, porous silicates) present to the extent of from 0.1% to 30% by weight of the combination. Other optional ingredients in the thermogenic material combination include oxidation reaction enhancers (chromium, manganese, copper), hydrogen gas inhibitors (sodium hydroxide, sodium thiosulfate), fillers (cellulosics), anti-caking agents (tricalcium phosphate, sodium silicoaluminates), thickeners (starches) and surfactants. Thermogenic materials of the type useful in the heating zone(s) of the devices herein are described in greater details in Burkett et al; U.S. Patent 5,918,590; Issued July 6, 1999.

Within the heating zone(s) of the devices herein, the thermogenic materials are separated or divided into at least two distinct regions. Upon activation of the dispensing device, the thermal energy in one such region of thermogenic materials must be produced at a faster rate and for a shorter period of time than thermal energy is produced in at least one other of the regions of the thermogenic materials.

One means for varying the thermal energy production within the regions of the thermogenic materials can be provided by varying the introduction rate of activating oxygen-containing gas which is brought into contact with the thermogenic materials in each region. This can be accomplished, for example, by varying the porosity of the thermogenic material mass from one region to another. Another way to accomplish variation in oxygen-containing gas introduction rate among regions is to vary the surface area of the mass of thermogenic materials within each region which is exposed to oxygen.

Variation in the surface area of the thermogenic materials exposed to oxygen-containing gas can be accomplished simply by varying the particle size of the thermogenic materials from region to region. Variation in thermogenic material surface area exposure can also be realized by placing the the thermogenic materials into first and second heating zones which have openable apertures therein of differing sizes in each zone. Such apertures, when opened, permit airflow into the first and second heating zones from the outside atmosphere. By varying the total cross-sectional area of the apertures in the first and second heating zones, variation of airflow into these zones can be realized. Thus, preferably the openable apertures in the first heating zone will have a total cross-sectional area which is from 10% to 700% larger than the total cross-sectional area of the apertures in the second heating zone. In preferred devices herein, the total cross-sectional area of the apertures in the first heating zone will range from 2.2 cm² to 14 cm², the total cross-sectional area of the apertures in the second heating zone will range from 2.0 cm² to 4.0 cm² and there will be a difference of at least 0.2 cm², preferably of at least 5 cm², in the total cross-sectional areas of the apertures in the first and second heating zones.

Another means for varying the thermal energy production within the regions of the thermogenic materials can be provided by varying the nature and makeup of the composition of the thermogenic materials among the different thermogenic material regions. Thus, for example, thermogenic material mixtures of different components can be employed in different regions. Different thermogenic material component concentrations and/or ratios among the different regions can also be used. Variation in water content among the thermogenic material regions is an especially preferred way of bringing about variation in thermal energy production rate.

Preferably, the several thermogenic material regions, for example the separate first and second or more heating zones, will be in thermodynamic communication with each other. In this manner, thermal energy generated quickly in the first region or heating zone can catalyze and promote the thermogenic reaction in the regions or zones with the slower initial energy production rates.

The thermogenic materials used in the first region or heating zone should, upon activation, be capable of producing a temperature within that region or heating zone of at least 60 °C within a period of from 3 to 7 minutes after contact of oxygen with the thermogenic materials is initiated. Preferably, this temperature will be maintained for a period of at least 25 minutes, more preferably at least 40 minutes, after activation of the device. The thermogenic materials in the second region or heating zone should, upon activation, be capable of producing a temperature within that region or heating zone of from 55 °C to 60 °C. Temperatures within this range should be maintained in this second region or heating zone for a period of at least 180 minutes, more preferably at least 240 minutes, after introduction of oxygen-containing gas begins.

The thermogenic materials in the heating zone(s) are preferably held and constrained within this zone by air-permeable holding means. Such holding means serves to prevent the generally solid, e.g., granular, thermogenic materials from being removed from the device, either purposefully or unintentionally, once the sealing means of the heating zone have been ruptured and the heating zone is thereby opened to the outside atmosphere. Such air-permeable holding means, can comprise, for example, fabric- or polymer-based mesh materials having mesh openings, e.g., apertures, therein which are large enough to permit suitable flow of air therethrough but small enough to prevent significant amounts of the solid thermogenic material from passing through the mesh.

### iii) Sealing Means

Both the volatilization zone and the heating zone(s) herein are preferably hermetically sealed from the outside atmosphere by sealing means. Such sealing means serve to prevent the escape of the volatilizable materials from the volatilization zone, and to prevent the activation of the thermogenic materials in the heating zone(s), until the sealing means are ruptured. The sealing means also preferably keep the contents of the volatilization zone and the heating zone(s) out of contact with each other.

Preferably the sealing means for both volatilization zone and the heating zone(s) will be located at more than one place on each zone. In a preferred configuration for the devices with inserts, wherein both the volatilization and heating zones are elongated and held in a generally vertical position, the rupturable sealing means will be positioned at both the upper and lower end of each zone. In this way, when the sealing means are ruptured at both ends of these zones, airflow into and through each zone is promoted.

Sealing means can comprise any kinds of materials which are both air and liquid impervious when intact but which can be readily ruptured when the dispensing device is to be activated. Suitable sealing means for both volatilization and heating zones include any type of single ply or laminated film, foil or sheet which has the requisite type of impermeability and rupturability characteristics. Laminated aluminum foil is especially preferred as a sealing means useful in the devices of this invention. The sealing material may, in fact be co-laminated with holding means for the thermogenic material, e.g., with flexible mesh. In this setup, the rupturing means of the holder may be configured to rupture the foil sealing means but not the flexible mesh holding means laminated to the foil.

### B. Holder

The inserts of the preferred device configuration, as described hereinbefore, are held with the volatilization chamber in a generally upright vertical position by a holder component for the insert. In the preferred devices herein, the holder comprises a substantially rigid base and a substantially rigid cover for the base. The shape of the holder cover and holder base will, of course, depend upon the shape and configuration of the insert which is to fit inside the holder. For inserts which are generally cylindrical in configuration, the base and cover comprising the holder will generally be cylindrical or spherical.

The holder element of the preferred devices herein must have means positioned on the cover or base, and preferably on both, for rupturing the sealing means used for the chambers or zones of the insert. Such rupturing means must be consumer activatable, i.e., when the user of the dispensing device wants to activate it, the user must be able to cause the rupturing means to puncture or rupture the sealing means on the insert zones so that exposure to air of the thermogenic materials begins the heat generation process and exposure to air of the volatilizable materials begins the process of dispensing these materials into the surrounding atmosphere. Generally the seal rupturing means associated with the base and/or cover simply comprises rigid and relatively sharp protrusions which can be forced against the sealing means until rupture of the sealing means occurs.

In one preferred configuration, the covering base of the holder are slideably affixed to each other such as the user of the device can activate it by pushing the cover and base closer together. As illustrated in the drawing hereinafter, this action serves to push the puncturing protrusions which are part of both base and cover into and through the seals of the insert which is held inside the slideably closeable base and cover.

The cover and base elements of the holder may also be configured to control or alter the flow of air into and through the dispensing device once the seals have been ruptured. This can be accomplished by altering the size of the air inlet openings and/or the configuration and number of seal puncturing means. The cover and base elements of the holder may also be rotatable or otherwise moveable in relation to each other in order to vary the size of the air inlet openings.

The holder may also preferably be constructed of thermochromic material such that at least a portion of the outside surface of the holder changes color upon heating. In this manner, the device can be constructed of a material which changes color when the device is activated and the holder heats up as a result of thermal energy generation in the heating zone(s). This provides a signal to the user of the device that the device has been activated and is generating heat and emitting volatiles.

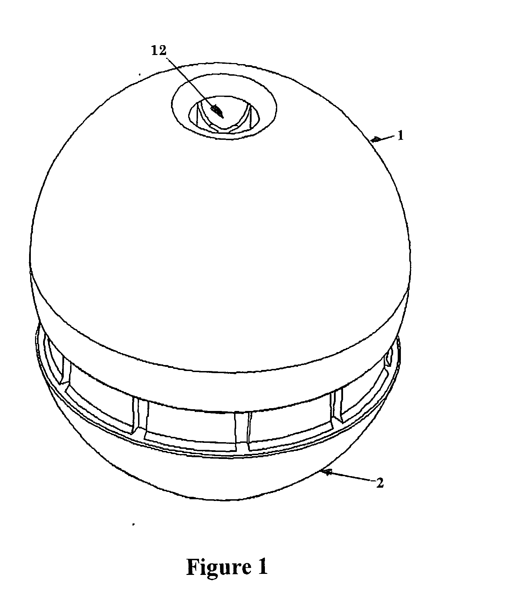

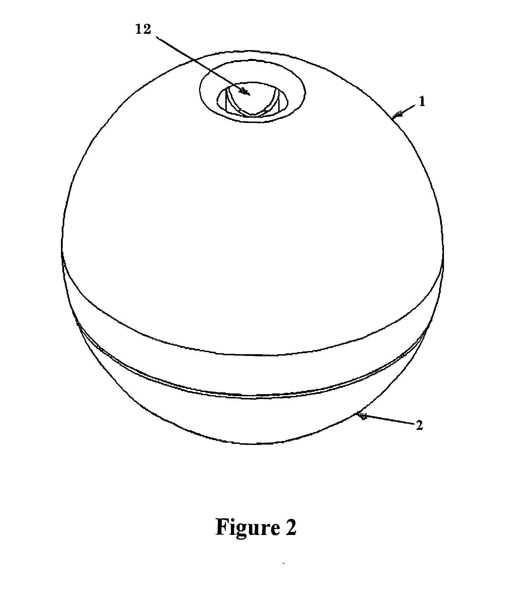

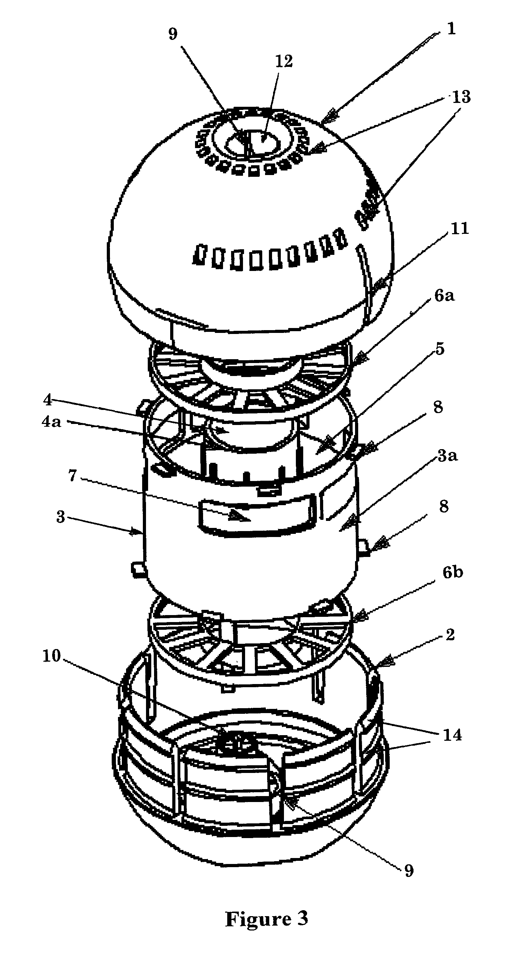

A highly preferred dispensing device according to the present invention is one having a cylindrical insert held within a spherical holder. Such a device depicted in Figures 1-3 of the drawings. Figure 1 is an external view of a dispensing device (without air holes) in its preactivated condition. Figure 2 is an external view of this same dispensing device which has been activated by pushing the holder pieces together. Figure 3 is an exploded view of a similar dispensing device which also has air holes in its cover.

In the several figures there is shown the dispensing device holder comprising a cover **1** which snaps onto a base **2.** Inside the cover and base is a generally cylindrical insert **3.** The insert comprises a cylindrical volatilization zone **4** along the axis of the insert. This volatilization zone contains volatilizable material (not shown) such as fragrance to be dispensed from the device.

The volatilization zone is surrounded by an annular chamber comprising a heating zone as defined by the wall **4a** of the volatilization zone and outside cylindrical wall **3a** of the insert. The heating zone is divided into an upper and a lower chamber by horizontal separating wall **5.** Positioned within both the upper and lower chambers of the annular heating zone is an effective amount of thermogenic materials (not shown) which are held in place within the chambers of the heating zone by upper and lower porous screens, **6a** and **6b,** respectively. Within the outside wall of the upper chamber of the heating zone are several air inlet openings **7.** (The outside wall of the lower chamber of the heating zone contains no such openings.) Prior to activation, these air inlet openings **7** in the upper chamber of the heating zone are covered with a strip of sealing tape (not shown). The end of this sealing tape strip protrudes through slot **11** in the upper cover **1.**

Prior to device activation, both the top and bottom of the insert are covered with sealing means (not shown), supported by the screens **6a** and **6b,** which seal the volatilization zone and the two chambers of the heating zone from the outside atmosphere. The sealed insert is provided with breakable support lugs **7** which support the insert within the base and cover of the holder before the device is activated. The cover and base are both provided with central top and bottom seal penetrating protrusions **9** which are positioned to rupture the top and bottom seals and open the volatilization zone at both of its ends. The cover and base are also provided with four perimeter top and bottom seal penetrating protrusions **10** (shown on bottom only) positioned to also puncture the top and bottom seals and open the heating zone. The base is also fitted with a bottom center air inlet opening (not shown) and other bottom perimeter air inlets (not shown) to provide exposure of the volatilization zone and heating zone, respectively, to the atmosphere upon rupture of the bottom seals when the user activated the device.

The cover also includes a top outlet opening **12** through which the volatile or volatilized materials are emitted to the outside atmosphere upon activation of the dispensing device. The base is also provided with a clip feature formed by ridges **14** into which the cover snaps. The clip features keep the cover and base apart, and the insert seals unpunctured, prior to activation of the device. Finally the cover also contains air holes **13** which permit flow of air into and through the heating zone once the device is activated.

### Mode of Operation

### Prior to activation:

The insert **3** is suspended inside the holder cover **1** and holder base **2.** The cover and base are kept apart to prevent rupturing of the insert seals by interacting with clip feature **14** and breakable support lugs **8.** A further anti-activation device (not shown in the drawings) could also be used to prevent movement of the cover and based towards each other. Such anti-activation devices could include a clip or shrink sleeve or tamper band.

### To activate:

The cover and base **1** and **2** are pushed together by overcoming the clip feature **14,** breaking or deforming lugs **8** and piercing the insert seals via the cutter protrusions **9** and **10.** The sealing tape is also removed from air inlet openings **7** by pulling it through slot **11.** These actions result in air being allowed to flow through the volatilization zone with the volatile, e.g., fragrance, materials therein and also into the two chambers of the heating zone. This results an exothermic reaction taking place in the heating zone.

The reaction is faster in the upper chamber of the heating zone than in the lower chamber because the upper chamber is exposed to more activating air. Heat generated quickly in the upper chamber of the heating zone then promotes heat generation in the slower reacting lower chamber of the heating zone. The upper chamber thus heats up quickly to a relatively high temperature, but the heat generation therein is of shorter duration than is the lower temperature heat generation in the slower reacting lower chamber. Heat generation from both chambers results in heat transfer from the heating zone into the volatilization zone. This, in turn, creates convection currents in the volatilization zone and enhances volatilization of the volatilizable materials which are discharged into the atmosphere through opening **12** at the top of the device.

## Claims

1. A dispensing device for emitting volatilized material into the atmosphere surrounding the device, said device being **characterized in that** it comprises:
A) at least one volatilization zone containing volatilizable material, said volatilization zone being openable to the outside atmosphere to permit emission of volatilized material therefrom, said volatilization zone further being juxtaposed with and in thermodynamic communication with;
B) one or more heating zones containing oxygen-activatable thermogenic materials capable of producing thermal energy via a non-combustive reaction when contacted with oxygen-containing gas, said thermogenic materials further being capable of transferring thermal energy so produced to said volatilization zone to thereby volatilize said volatilizable material therein;
wherein within said thermogenic materials in said heating zones there are at least two distinct regions wherein, upon activation of said device, thermal energy in one region is produced at a faster rate and for a shorter period of time than thermal energy is produced in at least one other of said regions.

2. A device according to Claim 1 wherein the variation in thermal energy production within the regions of thermogenic materials is provided by means for varying the introduction rates of oxygen-containing gas into contact with said thermogenic materials.

3. A device according to Claim 2 wherein said oxygen introduction rate variation means comprises variation in porosity within said regions of thermogenic materials.

4. A device according to Claims 2 or 3 wherein said oxygen introduction rate variation means comprises variation among regions in the surface area of said thermogenic materials exposed to oxygen-containing gas.

5. A device according to any of Claims 1 to 4 wherein the variation in thermal energy production within the regions of thermogenic materials is provided by variation in the composition of said thermogenic materials among said regions.

6. A device according to Claim 5 wherein the variation in thermal energy production within the regions of thermogenic materials is provided by variation in the concentrations and/or ratios of components within the composition of said thermogenic materials.

7. A device according to any of Claims 1 to 6 wherein said thermogenic materials comprise a combination of iron, carbon, water and inorganic salts.

8. A device according to Claim 7 wherein the variation in thermal energy production within the regions of thermogenic materials is provided by variation in the water content of said thermogenic materials among said regions.

9. A device according to any of Claims 1 to 8 wherein said regions of differing thermal energy production within said thermogenic materials are in thermodynamic communication with each other.

10. A dispensing device for emitting volatilized material into the atmosphere surrounding the device, said device being **characterized in that** it comprises:
A) a volatilization zone containing volatilizable material, said volatilization zone being openable to the outside atmosphere to permit emission of volatilized material therefrom, said volatilization zone further being juxtaposed with and in thermodynamic communication with;
B) both a first heating zone and a second heating zone, each of which heating zones contains oxygen-activatable thermogenic materials capable of producing thermal energy via a non-combustive reaction when contacted with oxygen-containing gas, said thermogenic materials further being capable of transferring thermal energy so produced to said volatilization zone to thereby volatilize said volatilizable material therein; and
C) means for introducing oxygen-containing gas into both first and second heating zones at introduction rates such that thermal energy is generated more rapidly and for a shorter period of time in said first heating zone than in said second heating zone.

11. A device according to Claim 10 wherein said introduction rates of said oxygen-containing gas are such that said thermogenic materials are capable of producing a temperature within said first heating zone of at least 60 ° C within a period of from 3 to 7 minutes after contact of air with said thermogenic materials is initiated.

12. A device according to Claim 10 or Claim 11 wherein said introduction rates of said oxygen-containing gas are such that the thermogenic materials in said second heating zone are capable of producing a temperature within said second heating zone of from 55 °C to 60 °C for a period of from 10 to 12 minutes after introduction of oxygen-containing gas begins.

13. A device according to any of Claims 10 to 12 wherein said gas introduction means comprises openable apertures in said first and second heating zones.

14. A dispensing device for emitting volatilized materials into the atmosphere surrounding the device, said device being **characterized in that** it comprises:
A) a volatilization zone containing volatilizable material, said volatilization zone being openable to the outside atmosphere to permit emission of volatilized material therefrom, said volatilization zone further being juxtaposed with and in thermodynamic communication with;
B) both a first heating zone and a second heating zone, each of which heating zones contains air-activatable thermogenic materials capable of producing thermal energy via a non-combustive reaction when contacted with air gas, said thermogenic materials further being capable of transferring thermal energy so produced to said volatilization zone to thereby volatilize said volatilizable material therein; and
C) openable apertures in both said first and second heating zones, said apertures being capable of permitting airflow into said first and second heating zones from the atmosphere surrounding said device, wherein the total cross-sectional area of said apertures in said first heating zone is from 10% to 700% larger than the total cross-sectional area of the apertures in said second heating zone.

15. A dispensing device according to Claim 14 wherein the total cross-sectional area of the apertures in said first heating zone ranges from 2.2 cm² to 14 cm² and wherein the total cross-sectional area of the apertures in said second heating zone ranges from 2.0 cm² to 4.0 cm² and wherein there is a difference of at least 0.2 cm² in the total cross-sectional areas of the apertures in said first and second heating zones.

16. A dispensing device for emitting volatile materials into the atmosphere surrounding the device, said device being **characterized in that** it comprises:
A) an insert comprising:
i) a substantially rigid volatilization zone containing volatile and/or volatilizable materials, said volatilization zone being juxtaposed with and in thermodynamic communication with:
ii) both a first heating zone and a second heating zone each of which contains air-activatable thermogenic materials;
both said volatilization zone and said first and second heating zones being sealed from the atmosphere surrounding said insert via sealing means which can be opened to expose all three zones to the atmosphere and to thereby activate the thermogenic materials within said heating zones and to thereby release volatile materials from the volatilization zone into the atmosphere; and
B) a holder for said insert, said holder comprising:
i) a substantially rigid base; and
ii) a substantially rigid cover for said base;
wherein said base and/or said cover of the holder comprise means for opening the sealing means of the volatilization and heating zones of said insert and
wherein the opening of said sealing means results in air introduction into said first heating zone at a faster rate than the air introduction into said second heating zone.

17. A device according to Claim 16 wherein said base and/or said cover of said holder can be moved in relation to said insert in a manner which causes the opening means associated with said base and/or cover to open the sealing means of said volatilization and heating zones of said insert.

18. A device according to Claim 16 or Claim 17 wherein the holder for the insert carries the insert in a manner which maintains the integrity of the sealing means for the zones of the insert until opening of said sealing means is desired.

19. A device according to any of Claims 16 to 18 wherein said opening means associated with said base and/or cover of said insert can be activated by the user of the device to open said sealing means of the volatilization and heating zones of said insert.

20. A device according to any of Claims 16 to 19 wherein said opening means comprise projections associated with said base and/or said cover of the holder, which projections are capable of puncturing said sealing means of the volatilization and heating zones of the insert upon the application of force onto said base and/or cover.

21. A device according to any of Claims 16 to 20 wherein said holder is constructed of thermochromic material such that at least a portion of the outside surface of said holder changes color when said device is activated and thermal energy is produced therein.

22. A device according to any of Claims 1 to 21 wherein the volatilizable materials within said volatilization zone are selected from the group consisting of fragrances, insect repellents, insecticides and materials which are or which produce therapeutic vapors.

23. A device according to any of Claims 9 to 22 wherein the thermogenic materials within said heating zones are capable of generating thermal energy via an iron oxidation reaction.

24. A device according to Claim 23 wherein said thermogenic materials comprise a combination of iron, carbon, water and inorganic salts.

25. A device according to any of Claims 9 to 24 wherein said first and second heating zones are in thermodynamic communication with each other.

26. Use of a volatiles dispensing device according to any of Claims 1 to 25 by initiating contact of said thermogenic materials within said device with air.
